# EUROPEAN PATENT SPECIFICATION

(11) **EP 2 793 853 B1**
(45) Date of publication and mention of the grant of the patent: **16.12.2015**
(21) Application number: 12823274.1
(22) Date of filing: 20.12.2012
(51) Int. Cl.: A61K 9/00, A61K 31/00, A61K 47/00, A61K 31/19, A61K 31/70

(54) **PHARMACEUTICAL FORMULATIONS OF FLURBIPROFEN AND GLUCOSAMIN**
PHARMAZEUTISCHE FORMULIERUNGEN AUS FLURBIPROFEN UND GLUCOSAMIN
FORMULATIONS PHARMACEUTIQUES DE FLURBIPROFÈNE ET GLUCOSAMINE

(30) Priority: 23.12.2011 TR 201112836; 31.01.2012 TR 201201091
(43) Date of publication of application: 29.10.2014
(73) Proprietor: Sanovel Ilac Sanayi Ve Ticaret Anonim Sirketi, 34460 Istanbul (TR)
(72) Inventor: CIFTER, Umit, 34460 Istanbul (TR); TURKYILMAZ, Ali, 34460 Istanbul (TR); MUTLU, Onur, 34460 Istanbul (TR)
(74) Representative: Sevinç, Erkan
(86) International application number: PCT/TR2012/000242
(87) International publication number: WO 2013/095317

(56) References cited:
- WO-A1-98/52545
- WO-A2-2008/008474
- US-A1- 2004 152 713
- US-A1- 2009 074 889
- DATABASE WPI Week 200681 Thomson Scientific, London, GB; AN 2006-792937 XP002681126, & JP 2006 290812 A (ROHTO SEIYAKU KK) 26 October 2006 (2006-10-26)

## Description

### Field of Invention

The present invention relates to pharmaceutical formulations of flurbiprofen or a pharmaceutically acceptable salt thereof and glucosamine or salts thereof. Particularly, the present invention relates to a stable formulation of this combination having desired levels of dissolution rate and solubility which comprises at least one polymer having a low glass transition temperature.

### Background of Invention

Osteoarthritis is the most prevalent form of arthritis, one of the most common diseases affecting humans and a common cause of disability. It is characterized by pain and progressive degeneration of cartilage in synovial joints and vertebrae, leading to significant reduction of mobility and quality of life. Hence, pharmacological treatment of arthritis involves two therapeutic goals:
- Analgesic & anti-inflammatory treatment: Relief from pain and inflammation of the soft tissue surrounding the joint.
- Disease-modifying treatment to treat the underlying pathology

Flurbiprofen is a well known, propionic acid derivative, also known as NSAID (non-steroidal anti-inflammatory drug), with the analgesic and anti-inflammatory activities it possesses. It is used in muscle-skeletal and joint disorders such as ankylosing spondylitis, osteoarthritis and rheumatoid arthritis, in soft-tissue disorders such as sprains and strains and for postoperative pains and mild to moderate pain including dysmenorrhoea and migraine. Its chemical structure is illustrated with Formula I given below.

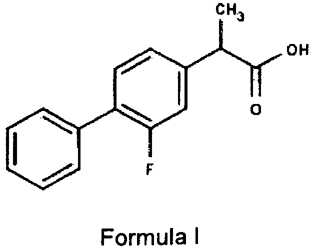

Flurbiprofen is mostly administrated orally in dosages about 150 to 200 mg, may also be increased to 300 mg daily in acute or severe conditions if necessary.

Glucosamine is an amino sugar and aprominent precursor in the biochemical synthesis of glycosylated proteins and lipids. Glucosamine is part of the structure of the polysaccharides chitosan and chitin and it is naturally present in the shells of shellfish, animal bones and bone marrow. It is also present in some fungi and can be also synthetically derived. The preferred salts of glucosamine include N-acetyl-glucosamine, glucosamine hydrochloride and glucosamine sulfate and mixtures thereof. Glucosamine is used for the treatment of osteoarthritis. Glucosamine may be administered in dosages about 500 to 2500 mg per day.

The solubility and dissolution rate of flurbiprofen may influence the bioavailability of glucosamine. For this reason, it is quite important to increase the solubility and dissolution rate of these active agents.

Another problem in relation to these active agents is stability, which emerges under the influence of ambient and physical conditions, as is the case with many other active agents. They are highly-susceptible to air and humidity. When they are exposed to air and humidity, they degrade structurally and develop chemical behavioral changes. The stability of the products developed may not at a desired level and the shelf life thereof may be shortened. In addition, these active agents are reactive against the excipients employed in developing the formulations containing the same. This, in turn, may cause impurities to occur in the formulations and may lead to the inclusion of undesired components into the formulations.

There are various patent applications in prior art in relation to glucosamine formulations but none of them are specifically used in combination with flurbiprofen in oral administration as tablet or capsule dosage form.

For example, US 2008/0227747 A1 discloses a therapeutic composition and methods for the treatment and prevention of a degenerative joint disorder and/or cardiovascular disease comprising polycosanols, glucosamine and chondroitin. Composition further may comprise NSAIDs, but neither an example nor flurbiprofen as one of the NSAIDs is disclosed in the patent application in combination with glucosamine, and the US application is silent about the problems related to its formulation and manufacturing process.

Another problem encountered while developing formulations of said active agents is the flowability-problem, which makes the manufacturing process difficult.

Another problem is related to combine these two active ingredients in one dosage form such as tablet or capsule, it would require a dosage form having approximately or more than 1000 mg active ingredients in total without any further tablet or capsule excipients. This is an amount that would create a very large tablet or capsule size that would not be swallowable, or it would require formulation that would require ingesting multiple tablets to achieve the desired effect. Therefore it has been found that in certain combinations with a specific polymer having a low glass transition temperature helps the formulation easily processed into a tablet or capsule dosage form, in desired weight which can easily be swallowed by the patients.

Finally, many of these techniques have proved to be successful only for specific drugs and are often not transferable to other active ingredients such as flurbiprofen in combination with glucosamine.

The object of the present invention is, therefore to provide stable, bioavailable, easily processed pharmaceutical formulations of flurbiprofen in combination with glucosamin by using at least one polymer having a low glass transition temperature, and to provide an alternative medicament for the treatment of osteoarthritis to those in the skilled art which overcomes the problems described above.

Further advantages and embodiments of the present invention will become apparent from the following description.

### Object and Summary of the Invention

The present invention relates to a stable, bioavailable, easily processed pharmaceutical formulations of flurbiprofen and glucosamin by using at least one polymer having a low glass transition temperature, for use in the treatment of osteoarthritis, pain and inflammatory symptoms associated with joint and cartilage disorders, which overcomes the above described problems in prior art and have additional advantages over them.

Accordingly, the main object of the present invention is to obtain a stable formulation with having a desired solubility and dissolution rate, and therefore a desired level of bioavailability.

A further object of the present invention is to provide easily processed pharmaceutical formulations of flurbiprofen and glucosamin produced by means of a hot-melt method by using at least one polymer having a low glass transition temperature, such as polyvinylcaprolactam-polyvinyl acetate-polyethylene glycol graft copolymer or stearyl macrogol glycerides.

A further object of the present invention is to eliminate the need for any liquid solvent, including water during the said process.

A further object of the present invention is to develop a formulation not leading to flowability-related problems during production.

A further object of the present invention is to obtain a uniform formulation content.

A pharmaceutical formulation is developed to carry out all objects, referred to above and to emerge from the following detailed description.

According to a preferred embodiment of the present invention, said novelty is realized with the formulation comprising,
a. flurbiprofen or pharmaceutically acceptable salts thereof,
b. glucosamine or salts thereof,
c. polyvinylcaprolactam-polyvinyl acetate-polyethylene glycol graft copolymer or stearyl macrogol glycerides.

According to another preferred embodiment of the present invention, said formulation is obtained by means of a hot-melt method not involving any liquid solvent during the granulation phase of flurbiprofen.

According to a preferred embodiment of the present invention, the weight ratio of flurbiprofen to polyvinylcaprolactam-polyvinyl acetate-polyethylene glycol graft copolymer or stearyl macrogol glycerides is in the range of 0.10 to 10.0, preferably 0.20 to 8.0, and more preferably 0.30 to 7.0.

According to a preferred embodiment of the present invention, the mean particle size (d₅₀) of the granules obtained by means of the hot-melt method is in the range of 100 - 1000 µm, preferably 300 - 800 µm, and more preferably 400 - 600 µm.

According to a preferred embodiment, the present invention also comprises polymers other than polyvinylcaprolactam-polyvinyl acetate-polyethylene glycol graft copolymer or stearyl macrogol glycerides which are selected from the group consists polyoxyethylene-polyoxypropylene block copolymers, cationic methacrylate, copovidone, methacrylic acid copolymer derivatives, cellulose acetate phthalate, acetylated monoglyceride, dibutyl tartrate, diethyl phthalate, dimethyl phthalate, glycerin, propylene glycol and tripropionin or their mixtures. The polymers having a low glass transition temperature are preferred in the present invention.

Another preferred embodiment of the present invention comprises at least one or more excipient. According to a preferred embodiment of the present invention, said excipient comprise at least one or more binders, disintegrants, glidants, lubricants, and plasticizers.

In a preferred embodiment of the present invention, said disintegrant is selected from a group comprising croscarmellose sodium, xylitol, polyplasdone (1-ethenylpyrrolidin-2-one), crospovidone, low-substituted hydroxypropyl cellulose (L-HPC) and sodium starch glycolate or mixtures thereof.

In a preferred embodiment of the present invention, said glidant is colloidal silicon dioxide or talc.

In a preferred embodiment of the present invention, said lubricant is selected from the group comprising magnesium stearate, sodium stearyl fumarate, polyethylene glycol, stearic acid, metal stearates, boric acid, sodium chloride benzoate and acetate, sodium or magnesium lauryl sulfate or mixtures thereof.

In a preferred embodiment of the present invention, said plasticizer is selected from the group comprising citrate esters such as acetyl tributyl citrate, acetyl triethyl citrate, or triethyl citrate; phthalate esters such as diethyl phthalate or dibutyl phthalate; fatty acid esters such as butly stearate, glycerol monostearate or stearyl alcohol; dibutyl sebacate, triacetine, castor oil, glycerin and low molecular weight polyethylene glycols or mixtures thereof, preferably the plasticizer is triacetine. The use of a plasticizer serves to reduce the glass transition temperature of the polymer and to increase the stability of active agents used in the formulation.

In a preferred embodiment according to the present invention, said pharmaceutical formulation consists of,
a. flurbiprofen or a pharmaceutically acceptable salt thereof at 6.0 - 11.0 % by weight,
b. glucosamine or salts thereof at 50.0 - 80.0 % by weight,
c. polyvinylcaprolactam-polyvinyl acetate-polyethylene glycol graft copolymer at 0.50 - 20.0 % by weight,
d. croscarmellose sodium at 1.0 - 10.0 % by weight,
e. colloidal silicon dioxide at 0.10 - 5.0 % by weight,
f. magnesium stearate at 0.10 - 5.0 % by weight,
g. triacetine at 0.10 - 10.0 % by weight.

In a preferred embodiment according to the present invention, said pharmaceutical formulation consists of,
a. flurbiprofen or a pharmaceutically acceptable salt thereof at 6.0 - 11.0 % by weight,
b. glucosamine or salts thereof at 50.0 - 80.0 % by weight,
c. stearyl macrogol glycerides at 0.50 - 20.0 % by weight,
d. croscarmellose sodium at 1.0 - 10.0 % by weight,
e. colloidal silicon dioxide at 0.10 - 5.0 % by weight,
f. magnesium stearate at 0.10 - 5.0 % by weight,
g. triacetine at 0.10 - 10.0 % by weight.

In another preferred embodiment of the present invention, said formulation is in the form of a tablet or capsule.

Another preferred embodiment according to the present invention provides a method for preparing said pharmaceutical formulation, this method comprising the steps of,
a. mixing flurbiprofen, polyvinylcaprolactam-polyvinyl acetate-polyethylene glycol graft copolymer and triacetine together, melting this mixture, and passing it through an extruder or sieve,
b. adding first glucosamine, croscarmellose sodium and colloidal silicon dioxide, and then magnesium stearate to the granules obtained and mixing the same,
c. performing a compression step on this powder mixture in a tablet machine, or filling this powder mixture into capsules.

Another preferred embodiment according to the present invention provides a method for preparing said pharmaceutical formulation, this method comprising the steps of,
a. mixing flurbiprofen, stearyl macrogol glycerides and triacetine together, melting this mixture, and passing it through a sieve or an extruder,
b. adding first glucosamine, croscarmellose sodium and colloidal silicon dioxide, and then magnesium stearate to the granules obtained and mixing the same,
c. performing a compression step on this powder mixture in a tablet machine, or filling this powder mixture into capsules.

According to another preferred embodiment of the present invention, the flurbiprofen or a pharmaceutically acceptable salts thereof combinations comprising glucosamine is used in the treatment of osteoarthritis, pain and inflammatory symptoms associated with joint and cartilage disorders.

Further advantages and embodiments of the present invention will become apparent from the following description

### Detailed Description of Invention

In this present invention, a stable formulation is surprisingly obtained which has a high solubility and dissolution rate. Said formulation comprises flurbiprofen and glucosamine sulfate and polyvinylcaprolactam-polyvinyl acetate-polyethylene glycol graft copolymer or stearyl macrogol glycerides. The method described below both serves to provide a uniform formulation content, and eliminates the need for any liquid solvent including water. Any flowability-related problems are also prevented with this production method. In said formulation, the weight ratio of flurbiprofen to polyvinylcaprolactam-polyvinyl acetate-polyethylene glycol graft copolymer or stearyl macrogol glycerides is in the range of 0.10 to 10.0 preferably 0.20 to 8.0, and more preferably 0.30 to 7.0. The effect of these ranges is to provide the desired dissolution rate and solubility. Polymers with low glass transition temperature and melting temperature are used in said formulation. On the other hand, using a plasticizer which reduces the glass transition temperature increases the stability of the active agent. The plasticizer used in a hot-melt method drops down the glass transition temperature of the polymers used in hot-melting, and thus allows to formulate the active agent at lower temperatures. In result, the formulation is made more stable.

According to the present invention, one of the preferred polymer which has a low glass transition temperature is polyvinylcaprolactam-polyvinyl acetate-polyethylene glycol graft copolymers or stearyl macrogol glycerides. Other poylmers are preferably selected from the group consisting of polyoxyethylene-polyoxypropylene block copolymers, cationic methacrylate, copovidone, methacrylic acid copolymer derivatives, cellulose acetate phthalate, acetylated monoglyceride, dibutyl tartrate, diethyl phthalate, dimethyl phthalate, glycerin, propylene glycol and tripropionin or their mixtures.

According to a further embodiment of the present invention, glucosamine may further be combined with chondroitin or methylsulfonylmethane.

The pharmaceutical compositions according to the present invention may also comprise one or more pharmaceutically acceptable excipients. Such pharmaceutically acceptable excipients include, but are not limited to binders, disintegrants, glidants, lubricants, plasticizers, surface active agents, preservatives and the mixtures thereof. It is also possible to use the following additional excipients in this formulation as shown below:
a. flurbiprofen or pharmaceutically acceptable salts thereof 6.0 - 11.0% by weight
b. glucosamine sulfate 50.0 - 80.0 % by weight
c. binder 0.50 - 20.0 % by weight
d. disintegrant 1.0 - 10.0 % by weight
e. lubricant 0.10 - 5.0 % by weight
f. glidant 0.10-5.0% by weight
g. plasticizer 0.10 - 10.0 % by weight.
h. preservatives 0.0 to 2.0 % by weight.
i. surface active agents 0.0 to 5.0 % by weight

Suitable binders, may include but not limited to polymetacrylate, polyvinylpyrrolidone (povidon), hydroxsypropyl methyl cellulose (HPMC), hydroxsypropyl cellulose (HPC), carboxsy methyl cellulose (CMC), methyl cellulose (MC), hydroxy ethyl cellulose, sodium carboxy methyl cellulose (NaCMC), carboxymethyl cellulose calsium, ethyl cellulose, polyethylene oxide, gelatin, starch, xanthan gum, guar gum, alginate, carrageenan, pectin, carbomer, cellulose acetat phytalate, hydroxy propyl starch, polaxomer, poly ethylene glychol or mixtures thereof.

Suitable disintegrants, may include but not limited to croscarmellose sodium, xylitol, polyplasdone (1-ethenylpyrrolidin-2-one), crospovidone, low-substituted hydroxypropyl cellulose (L-HPC) and sodium starch glycolate or mixtures thereof.

Suitable lubricants, may include but not limited to magnesium stearate, sodium stearyl fumarate, polyethylene glycol, stearic acid, metal stearates, boric acid, sodium chloride benzoate and acetate, sodium or magnesium lauryl sulfate or mixtures thereof.

Suitable glidants, may include but not limited to colloidal silicon dioxide or talc or mixtures thereof.

Suitable preservatives, may include but not limited to methyl paraben, propyl paraben and salts thereof (e.g. sodium or potassium salts), sodium benzoate, citric acid, benzoic acid, butylated hydroxytoluene and butylated hydroxyanisole or mixtures thereof.

Suitable surface active agents, may include but not limited to dioctyl sulfosuccinate, polysorbates and polyoxyethylene alkyl esters and ethers thereof, glyceryl monolaurate saponins, sorbitan laurate, sodium lauryl sulfate, magnesium lauryl sulfate or mixtures thereof.

This invention is further defined by reference to the following examples. Although the example is not intended to limit the scope of the present invention, it should be considered in the light of the description detailed above. It will be apparent to those skilled in the art that many modifications, both to materials and methods, may be practiced without departing from the scope of the invention.

### Example 1: capsul or tablet

| **Ingredients** | **% amount (mg)** |
|---|---|
| flurbiprofen | 6.0 - 11.0 |
| glucosmain or salts thereof | 50.0 - 80.0 |
| polyvinylcaprolactam-polyvinyl acetate-polyethylene glycol graft copolymer | 0.50 - 20.0 |
| croscarmellose sodium | 1.0 - 10.0 |
| colloidal silicon dioxide | 0.10 - 5.0 |
| magnesium stearate | 0.10 - 5.0 |
| triacetine | 0.10- 10.0 |

The proses of the formulation is carried out as follows: Flurbiprofen, triacetine and polyvinylcaprolactam-polyvinyl acetate-polyethylene glycol graft copolymer are mixed together, this mixture is melted and passed through an extruder or sieve. First glucosamine croscarmellose sodium and colloidal silicon dioxide are added, and then magnesium stearate is added to the granules obtained and the resulting mixture is mixed. A compression step is performed on this powder mixture in a tablet machine, or this powder mixture is filled into capsules. The tablets are coated preferably with a humidity-barrier coating material, such as Kollicoat IR or Opadry Amb white which comprises polyvinyl alcohol, titanium dioxide, talc, lecitine, xantan guar.

### Example 2: capsul or tablet

| **Ingredients** | **% amount (mg)** |
|---|---|
| flurbiprofen | 6.0 - 11.0 |
| glucosamine or salts thereof | 50.0 - 80.0 |
| stearyl macrogol glycerides | 0.50 - 20.0 |
| croscarmellose sodium | 1.0 - 10.0 |
| colloidal silicon dioxide | 0.10 - 5.0 |
| magnesium stearate | 0.10 - 5.0 |
| triacetine | 0.10 -10.0 |

The proses of the formulation is carried out as follows: Flurbiprofen, triacetine and stearyl macrogol glycerides are mixed together, this mixture is melted and passed through an extruder or sieve. First glucosamine, croscarmellose sodium and colloidal silicon dioxide are added, and then magnesium stearate is added to the granules obtained and the resulting mixture is mixed. A compression step is performed on this powder mixture in a tablet machine, or this powder mixture is filled into capsules. The tablets are coated preferably with a humidity-barrier coating material, such as Kollicoat IR or Opadry amb white which comprises polyvinyl alcohol, titanium dioxide, talc, lecitine, xantan guar.

## Claims

1. A pharmaceutical formulation, comprising
a. flurbiprofen or pharmaceutically acceptable salts thereof,
b. glucosamine or salts thereof,
c. polyvinylcaprolactam-polyvinyl acetate-polyethylene glycol graft copolymer or stearyl macrogol glycerides.

2. The pharmaceutical formulation according to claim 1, wherein said formulation is obtained by means of a hot-melt method and not involving any liquid solvent during the granulation phase of flurbiprofen.

3. The pharmaceutical formulation according to claim 1, wherein the weight ratio of flurbiprofen to polyvinylcaprolactam-polyvinyl acetate-polyethylene glycol graft copolymer or stearyl macrogol glycerides is in the range of 0.10 to 10.0, preferably 0.20 to 8.0, and more preferably 0.30 to 7.0.

4. The pharmaceutical formulation according to claims 1 to 3, wherein the mean particle size (d₅₀) of the granules obtained by means of the hot-melt method is in the range of 100 - 1000 µm, preferably 300 - 800 µm, and more preferably 400 - 600 µm.

5. The pharmaceutical formulation according to claim 1, further comprising polymers other than polyvinylcaprolactam-polyvinyl acetate-polyethylene glycol graft copolymer or stearyl macrogol glycerides which are selected from the group consisting of polyoxyethylene-polyoxypropylene block copolymers, cationic methacrylate, copovidone, methacrylic acid copolymer derivatives, cellulose acetate phthalate, acetylated monoglyceride, dibutyl tartrate, diethyl phthalate, dimethyl phthalate, glycerin, propylene glycol and tripropionin or their mixtures.

6. The pharmaceutical formulation according to claims 1 to 5, further comprising at least one or more excipient.

7. The pharmaceutical formulation according to claim 6, wherein said excipient comprises at least one or more binders, disintegrants, glidants, lubricants, and plasticizers.

8. The pharmaceutical formulation according to claim 7, wherein said disintegrant is selected from a group comprising croscarmellose sodium, xylitol, polyplasdone (1-ethenylpyrrolidin-2-one), crospovidone, low-substituted hydroxypropyl cellulose (L-HPC) and sodium starch glycolate or mixtures thereof.

9. The pharmaceutical formulation according to claim 7, wherein said glidant is colloidal silicon dioxide or talc.

10. The pharmaceutical formulation according to claim 7, wherein said lubricant is selected from the group comprising magnesium stearate, sodium stearyl fumarate, polyethylene glycol, stearic acid, metal stearates, boric acid, sodium chloride benzoate and acetate, sodium or magnesium lauryl sulfate or mixtures thereof.

11. The pharmaceutical formulation according to claim 7, wherein said plasticizer is selected from the group comprising citrate esters such as acetyl tributyl citrate, acetyl triethyl citrate or triethyl citrate; phthalate esters such as diethyl phthalate or dibutyl phthalate; fatty acid esters such as butly stearate, glycerol monostearate or stearyl alcohol; dibutyl sebacate, triacetine, castor oil, glycerin and low molecular weight polyethylene glycols or mixtures thereof, preferably the plasticizer is triacetine.

12. The pharmaceutical formulation according to any of the preceding claims, consisting of,
a. flurbiprofen or a pharmaceutically acceptable salt thereof at 6.0 - 11.0 % by weight,
b. glucosmain or salts thereof at 50.0 - 80.0 % by weight,
c. polyvinylcaprolactam-polyvinyl acetate-polyethylene glycol graft copolymer at 0.50 - 20.0 % by weight,
d. croscarmellose sodium at 1.0 -10.0 % by weight,
e. colloidal silicon dioxide at 0.10 - 5.0 % by weight,
f. magnesium stearate at 0.10 - 5.0 % by weight,
g. triacetine at 0.10 - 10.0% by weight.

13. The pharmaceutical formulation according to any of the preceding claims, consisting of,
a. flurbiprofen or a pharmaceutically acceptable salt thereof at 6.0 - 11.0 % by weight,
b. glucosmain or salts thereof at 50.0 - 80.0 % by weight,
c. stearyl macrogol glycerides at 0.50 - 20.0 % by weight,
d. croscarmellose sodium at 1.0 -10.0 % by weight,
e. colloidal silicon dioxide at 0.10 - 5.0 % by weight,
f. magnesium stearate at 0.10 - 5.0 % by weight,
g. triacetine at 0.10 - 10.0% by weight.

14. The pharmaceutical formulation according to any of the preceding claims, wherein the formulation is in the form of a tablet or capsule.

15. A method for preparing a pharmaceutical formulation according to claims 12 and 14, comprising the steps of
a. mixing flurbiprofen, triacetine and polyvinylcaprolactam-polyvinyl acetate-polyethylene glycol graft copolymer together, melting this mixture, and passing it through an extruder or sieve,
b. adding first glucosamine, croscarmellose sodium and colloidal silicon dioxide, and then magnesium stearate to the granules obtained and mixing the same,
c. performing a compression step on this powder mixture in a tablet machine, or filling this powder mixture into capsules.

## Patentansprüche

1. Pharmazeutische Formulierung, umfassend:
a. Flurbiprofen oder dessen pharmazeutisch unbedenklichen Salze,
b. Glucosamin oder dessen Salze,
c. Polyvinylcaprolactam-Polyvinylacetat-Polyethylenglycol-Propfcopolymer oder Stearyl-Macrogolglyceride.

2. Pharmazeutische Formulierung nach Anspruch 1, bei der diese Formulierung durch ein Hotmelt-Verfahren ohne Einbeziehung eines flüssigen Lösungsmittels während der Granulationsphase von Flurbiprofen erhalten wird.

3. Pharmazeutische Formulierung nach Anspruch 1, wobei das Gewichtsverhältnis von Flurbiprofen zu Polyvinylcaprolactam-Polyvinylacetat-Polyethylenglycol-Propfcopolymer oder Stearyl-Macrogolglyceriden im Bereich von 0,10 bis 10,0, vorzugsweise von 0,20 bis 8,0, und noch bevorzugter von 0,30 bis 7,0 liegt.

4. Pharmazeutische Formulierung nach den Ansprüchen 1 bis 3, bei der die mittlere Teilchengröße (d₅₀) des mit dem Hotmelt-Verfahren erhaltenen Granulats im Bereich von 100-1000 µm, vorzugsweise von 300-800 µm, und noch bevorzugter von 400-600 µm liegt.

5. Pharmazeutische Formulierung nach Anspruch 1, des weiteren umfassend andere Polymere als Polyvinylcaprolactam-Polyvinylacetat-Polyethylenglycol-Propfcopolymer oder Stearyl-Macrogolglyceride, welche aus der Gruppe mit Polyoxyethylen-Polyoxypropylen-Blockcopolymeren, kationischem Methacrylat, Copovidon, Methacrylsäurecopolymerderivate, Celluloseacetatphthalat, acetyliertem Monoglycerid, Dibutyltartrat, Diethylphthalat, Dimethylphthalat, Glycerin, Propylenglycol und Tripropionin oder Mischungen daraus ausgewählt sind.

6. Pharmazeutische Formulierung nach den Ansprüchen 1 bis 5, des Weiteren umfassend zumindest einen oder mehrere Arzneiträger.

7. Pharmazeutische Formulierung nach Anspruch 6, bei der der Arzneiträger mindestens ein oder mehrere Bindemittel, Sprengmittel, Fließregulierungsmittel, Schmiermittel und Weichmacher umfasst.

8. Pharmazeutische Formulierung nach Anspruch 7, bei der das Sprengmittel ausgewählt ist aus einer Gruppe, welche Croscarmellose-Natrium, Xylitol, Polyplasdon (1-Ethenylpyrrolidin-2-on), Crospovidon, niedrig substituierte Hydroxypropylcellulose (L-HPC) und Natriumstärkeglykolat oder Mischungen daraus umfasst.

9. Pharmazeutische Formulierung nach Anspruch 7, bei der das Fließregulierungsmittel kolloidales Siliciumdioxid oder Talk ist.

10. Pharmazeutische Formulierung nach Anspruch 7, bei der das Schmiermittel ausgewählt ist aus der Gruppe, welche Magnesiumstearat, Natriumstearylfumarat, Polyethylenglycol, Stearinsäure, Metallstearate, Borsäure, Natriumchloridbenzoat und -acetat, Natrium- oder Magnesiumlaurylsulfat oder Mischungen daraus umfasst.

11. Pharmazeutische Formulierung nach Anspruch 7, bei der der Weichmacher ausgewählt ist aus der Gruppe umfassend Citratester, beispielsweise Acetyltributylcitrat, Acetyltriethylcitrat oder Triethylcitrat; Phthalatester, beispielsweise Diethylphthalat oder Dibutylphthalat; Fettsäureester, beispielsweise Butylstearat, Glycerolmonostearat oder Stearylalkohol; Dibutylsebacat, Triacetin, Castoröl, Glycerin und Polyethylenglycole mit niedrigem Molekulargewicht sowie Mischungen daraus, wobei der Weichmacher bevorzugt Triacetin ist.

12. Pharmazeutische Formulierung nach einem der vorhergehenden Ansprüche, bestehend aus:
a. Flurbiprofen oder eines seiner pharmazeutisch unbedenklichen Salze zu 6,0-11,0 Gew.-%,
b. Glucosamin oder dessen Salze zu 50,0 - 80,0 Gew.-%,
c. Polyvinylcaprolactam-Polyvinylacetat-Polyethylenglycol-Propfcopolymer zu 0,50 - 20,0 Gew.-%,
d. Croscarmellose-Natrium zu 1,0 - 10,0 Gew.-%,
e. kolloidales Siliciumdioxid zu 0,10 - 5,0 Gew.-%,
f. Magnesiumstearat zu 0,10 - 5,0 Gew.-%,
g. Triacetin zu 0,10 bis 10,0 Gew.-%.

13. Pharmazeutische Formulierung nach einem der vorhergehenden Ansprüche, bestehend aus:
a. Flurbiprofen oder eines seiner pharmazeutisch unbedenklichen Salze zu 6,0-11,0 Gew.-%,
b. Glucosamin oder dessen Salze zu 50,0 - 80,0 Gew.-%,
c. Stearyl-Macrogolglyceride zu 0,50 - 20,0 Gew.-%,
d. Croscarmellose-Natrium zu 1,0 - 10,0 Gew.-%,
e. kolloidales Siliciumdioxid zu 0,10 - 5,0 Gew.-%,
f. Magnesiumstearat zu 0,10 - 5,0 Gew.-%,
g. Triacetin zu 0,10 bis 10,0 Gew.-%.

14. Pharmazeutische Formulierung nach einem der vorhergehenden Ansprüche, bei der die Formulierung in Tabletten- oder Kapselform vorliegt.

15. Verfahren zur Herstellung einer pharmazeutischen Formulierung nach den Ansprüchen 12 und 14, folgende Schritte umfassend:
a. Vermischen von Flurbiprofen, Triacetin und Polyvinylcaprolactam-Polyvinylacetat-Polyethylenglycol-Propfcopolymer, Schmelzen dieses Gemisches und Leiten des Gemisches durch einen Extruder oder ein Sieb,
b. Zugabe von zunächst Glucosamin, Croscarmellose-Natrium und kolloidalem Siliciumdioxid, und anschließend Magnesiumstearat zum erhaltenen Granulat und Vermischen,
c. Durchführen eines Verdichtungsschrittes dieses Pulvergemisches in einer Tablettenpressmaschine, oder Befüllen von Kapseln mit diesem Pulvergemisch.

## Revendications

1. Formulation pharmaceutique, comprenant
a. du flurbiprofène ou des sels pharmaceutiquement acceptables de celui-ci,
b. de la glucosamine ou des sels de celle-ci,
c. un copolymère greffé polyvinylcaprolactame-acétate de polyvinyle-polyéthylène glycol ou des stéaroyl macrogolglycérides.

2. Formulation pharmaceutique selon la revendication 1, dans laquelle ladite formulation est obtenue à l'aide d'un procédé de thermofusion et ne nécessitant aucun solvant liquide lors de la phase de granulation du flurbiprofène.

3. Formulation pharmaceutique selon la revendication 1, dans laquelle le rapport pondéral du flurbiprofène au copolymère greffé polyvinylcaprolactame-acétate de polyvinyle-polyéthylène glycol ou aux stéaroyl macrogolglycérides est compris entre 0,10 et 10,0, préférentiellement entre 0,20 et 8,0, et plus préférentiellement entre 0,30 et 7,0.

4. Formulation pharmaceutique selon les revendications 1 à 3, dans laquelle la taille de particule moyenne (d₅₀) des granulés obtenus à l'aide du procédé de thermofusion est comprise entre 100 et 1000 µm, préférentiellement entre 300 et 800 µm, et plus préférentiellement entre 400 et 600 µm.

5. Formulation pharmaceutique selon la revendication 1, comprenant en outre des polymères autres qu'un copolymère greffé polyvinylcaprolactame-acétate de polyvinyle-polyéthylène glycol ou des stéaroyl macrogolglycérides qui sont sélectionnés parmi le groupe constitué de copolymères séquencés de polyoxyéthylène-polyoxypropylène, méthacrylate cationique, copovidone, dérivés de copolymères d'acide méthacrylique, acétate phtalate de cellulose, monoglycéride acétylé, tartrate de dibutyle, phtalate de diéthyle, phtalate de diméthyle, glycérine, propylène glycol et tripropionine, ou leurs mélanges.

6. Formulation pharmaceutique selon les revendications 1 à 5, comprenant en outre au moins un ou plusieurs excipients.

7. Formulation pharmaceutique selon la revendication 6, dans laquelle ledit excipient comprend au moins un ou plusieurs liants, agents désintégrants, agents de glissement, lubrifiants et plastifiants.

8. Formulation pharmaceutique selon la revendication 7, dans laquelle ledit agent désintégrant est sélectionné parmi un groupe comprenant du sodium croscarmellose de sodium, du xylitol, de la polyplasdone (1-ethenylpyrrolidin-2-one), de la crospovidone, de l'hydroxypropylcellulose faiblement substituée (L-HPC) et du glycolate d'amidon sodique ou des mélanges de ceux-ci.

9. Formulation pharmaceutique selon la revendication 7, dans laquelle ledit agent de glissement est du dioxyde de silicium colloïdal ou du talc.

10. Formulation pharmaceutique selon la revendication 7, dans laquelle ledit lubrifiant est sélectionné parmi le groupe comprenant le stéarate de magnésium, le stéarylfumarate de sodium, le polyéthylène glycol, l'acide stéarique, les stéarates métalliques, l'acide borique, les benzoate et acétate de chlorure de sodium, le laurylsulfate de sodium ou de magnésium ou des mélanges de ceux-ci.

11. Formulation pharmaceutique selon la revendication 7, dans laquelle ledit plastifiant est sélectionné parmi le groupe comprenant des esters de citrate comme l'acétylcitrate de tributyle, l'acétylcitrate de triéthyle ou le citrate de triéthyle ; des esters de phtalate comme le phtalate de diéthyle ou le phtalate de dibutyle ; des esters d'acides gras comme le stéarate de butyle, le monostéarate de glycérol ou l'alcool stéarylique ; du dibutyl sébacate, de la triacétine, de l'huile de ricin, de la glycérine et des polyéthylènes glycols de faible poids moléculaire ou des mélanges de ceux-ci, préférentiellement le plastifiant est de la triacétine.

12. Formulation pharmaceutique selon l'une quelconque des revendications précédentes, consistant en,
a. du flurbiprofène ou un sel pharmaceutiquement acceptable de celui-ci à raison de 6,0 à 11,0 % en poids,
b. de la glucosamine ou des sels de celle-ci à raison de 50,0 à 80,0 % en poids,
c. un copolymère greffé polyvinylcaprolactame-acétate de polyvinyle-polyéthylène glycol à raison de 0,50 à 20,0 % en poids,
d. du croscarmellose de sodium à raison de 1,0 à 10,0 % en poids,
e. du dioxyde de silicium colloïdal à raison de 0,10 à 5,0 % en poids,
f. du stéarate de magnésium à raison de 0,10 à 5,0 % en poids,
g. de la triacétine à raison de 0,10 à 10,0 % en poids.

13. Formulation pharmaceutique selon l'une quelconque des revendications précédentes, consistant en,
a. du flurbiprofène ou un sel pharmaceutiquement acceptable de celui-ci à raison de 6,0 à 11,0 % en poids,
b. de la glucosamine ou des sels de celle-ci à raison de 50,0 à 80,0 % en poids,
c. des stéaroyl macrogolglycérides à raison de 0,50 à 20,0 % en poids,
d. du croscarmellose de sodium à raison de 1,0 à 10,0 % en poids,
e. du dioxyde de silicium colloïdal à raison de 0,10 à 5,0 % en poids,
f. du stéarate de magnésium à raison de 0,10 à 5,0 % en poids,
g. de la triacétine à raison de 0,10 à 10,0 % en poids.

14. Formulation pharmaceutique selon l'une quelconque des revendications précédentes, dans laquelle la formulation est sous la forme d'un comprimé ou d'une capsule.

15. Procédé de préparation d'une formulation pharmaceutique selon les revendications 12 et 14, comprenant les étapes qui consistent à
a. mélanger le flurbiprofène, la triacétine et le copolymère greffé polyvinylcaprolactame-acétate de polyvinyle-polyéthylène glycol ensemble, faire fondre ce mélange et le passer dans une extrudeuse ou un tamis,
b. ajouter d'abord la glucosamine, le croscarmellose de sodium et le dioxyde de silicium colloïdal, et ensuite le stéarate de magnésium aux granulés obtenus puis mélanger,
c. effectuer une étape de compression sur ce mélange de poudre dans une machine à comprimés, ou remplir les capsules avec ce mélange de poudre.
